# EUROPEAN PATENT SPECIFICATION

(11) **EP 0 599 429 B1**
(45) Date of publication and mention of the grant of the patent: **22.10.1997**
(21) Application number: 93203304.6
(22) Date of filing: 26.11.1993
(51) Int. Cl.: A61F 2/36, A61F 2/34, A61F 2/32

(54) **Femoral part of a hip joint endoprosthesis**
Femurteil einer Hüftgelenksprothese
Partie fémorale d'une endoprothèse joint avec la hanche

(30) Priority: 26.11.1992 DE 9216094 U
(43) Date of publication of application: 01.06.1994
(73) Proprietor: Bristol-Myers Squibb Company, New York, N.Y. 10154 (US)
(72) Inventor: Schelhas, Klaus-Dieter, D-2833 Harpstedt (DE)
(74) Representative: Mays, Julie

(56) References cited:
- US-A- 5 002 581
- US-A- 5 030 234
- US-A- 5 108 451

## Description

This invention concerns a femoral part of a hip joint endoprosthesis having a shaft, a spherical head and a connecting part connecting the shaft to the head, with a conical recess being provided on the proximal end of the shaft, a cooperating first conical plug on the end of the connecting part nearest the shaft and a second conical plug on the end of the connecting part nearest the head which projects into a cooperating conical recess on the head.

Such a femoral part where the spherical head is connected to the shaft by a connecting part is known from German Patent Application P 36 00 804.4, where the individual parts are joined together by means of a conical plug connector on the shaft side and on the head side. In order to be able to achieve the desired angle required for each patient and to permit an alignment of the head relative to the shaft that is suitable for the patient, the longitudinal axis of the first conical plug and the longitudinal axis of the second conical plug intersect at a given angle. A hip joint endoprosthesis that is adapted to the individual patient's needs should be obtainable through a suitable choice of the angled connecting part and an appropriate alignment of the head relative to the shaft. However, it has been found that especially those configurations of the natural hip joint where there is a great distance between the acetabulum of the pelvic bone and the axis of the femoral cannot be adequately simulated with this known femoral part of the known hip joint endoprosthesis.

Therefore, the object of this invention is to improve on the femoral part of known type such that hip joints that involve a relatively great distance between the acetabulum and the femoral axis can also be simulated accurately.

This object is achieved according to this invention with the femoral part of the type described above due to the fact that the longitudinal axis of the second conical plug has a parallel offset with respect to the longitudinal axis of the first conical plug.

The advantages of this invention include especially the fact that the parallel offset between the longitudinal axes of the two conical plugs of the connecting part allow implementation of a comparatively great distance between the center of the head and the axis of the shaft that is aligned with the axis of the femoral when implanted. Since the length of the conical plugs can also be adapted to the situation in the natural hip joint, the distance from the center of the head to the axis of the shaft on the one hand and to the plane of resection on the other hand can be adapted well to the given patient's needs. This especially makes it possible to simulate hip joints where the distance between the acetabulum of the pelvic bone and the axis of the femoral is comparatively great. In patients in whom the femoral is thus greatly lateralized, a good prosthetic simulation of the natural bone structure can thus be achieved by means of the femoral part according to this invention.

The connecting part can be secured especially well relative to the shaft by means of fastening devices in order to prevent rotation of the connecting part after implantation. Fastening devices that can be provided include attachments on the connecting part or on the shaft which engage in a corresponding recess in the shaft or the connecting part in a form-fitting manner in given relative positions.

According to a preferred embodiment of this invention, the first conical plug can be provided with a non-round cross section and the recess in the shaft may have a matching non-round cross section in order to secure the connecting part in the shaft in such a way that it cannot rotate. As an alternative attachment device, a polygonal connecting part that can project into a corresponding polygonal recess in the shaft in a form-fitting manner in given angular positions may be provided.

According to an especially preferred embodiment of this invention, several connecting parts with a different offset but with a constant length of the conical plugs may be kept on hand for one femoral part, so the surgeon can reproduce the correct distance between the center of the head and the axis of the femoral during the operation - simply by selecting the proper connecting part. In addition, it is also possible according to this invention to make available connecting parts with conical plugs of different lengths so the surgeon can also create the proper distance between the center of the head and the plane of resection - simply by selecting a suitable connecting part.

If desired, the conical plugs of the connecting part can also be angled with respect to each other in addition to the offsetting of the axes, so the longitudinal axes will intersect at a given angle.

Other advantageous embodiments of this invention are characterized by the features according to the subclaims.

One embodiment of this invention will be explained in greater detail below with reference to the figures.

This figure shows a femoral part 1 of a hip joint endoprosthesis with a shaft 2, a connecting part 10 and a head 20. The distal end of the shaft is cut away in the diagram shown here. The proximal end of the shaft has a collar 4 running around it. A conical recess 8 that extends into the shaft 2 to a point below the collar is cut from the proximal surface of the collar 4 - at a given angle α to the axis 3 of the shaft.

A first conical plug 12 on the end of connecting part 10 near the shaft fits into the conical recess 8 on the proximal end of the shaft 2. A central section 16 is connected to the conical plug 12 of the connecting part 10 and a second conical plug 14 that forms the end of the connecting part 10 near the head is in turn connected to it and can be anchored in a conical recess 18 on head 20. The central section 16 in the embodiment illustrated here is designed as a polygon that can be inserted into a matching polygonal recess 6 on the proximal surface of neck 4 in several given angular positions.

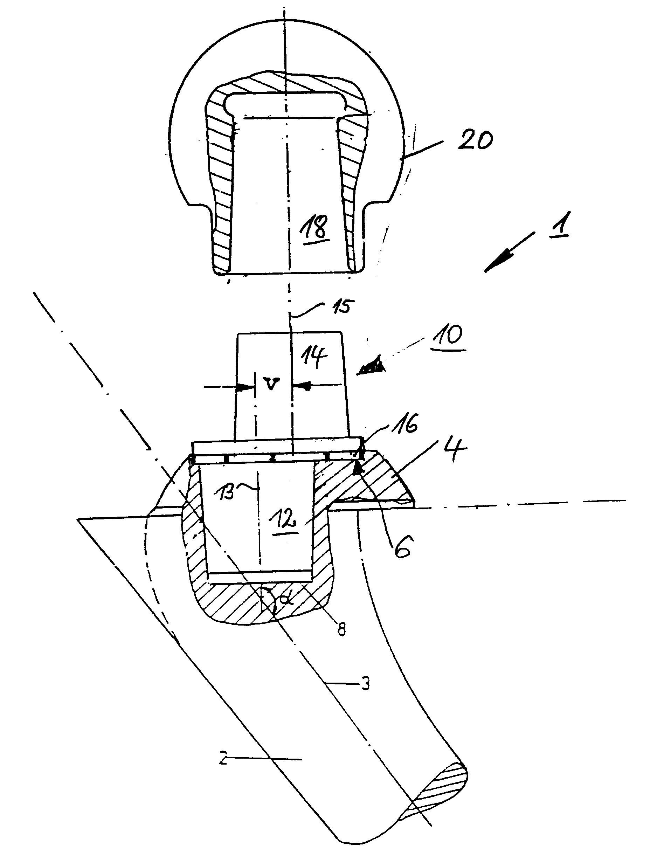

As shown clearly in the figure, the second conical plug 14 has an offset relative to the axis of the first conical plug 12 of the connecting part 10 - specifically, the longitudinal axis 15 of the second conical plug 14 is offset medially by the distance v with respect to the longitudinal axis 13 of the first conical plug 12 in order to implement a relatively great distance between the center point of head 20 and the axis 3 of the shaft.

If several connecting parts 10, each having a constant length of the conical plugs 14 and different offsets v, are kept on hand, the distance between the midpoint of the head and the axis 3 of the shaft can be implemented through a suitable choice of the connecting part in a desired manner while maintaining a constant distance between the midpoint of the head and the plane of resection.

## Claims

1. Femoral part of a hip joint endoprosthesis, said part comprising a shaft (2), a spherical head (20) and a connecting part (10) connecting the shaft to the head, with a conical recess being provided on the proximal end of the shaft, a first cooperating conical plug (12) on the end of the connecting part nearest the shaft and a second conical plug (14) on the end of the connecting part nearest the head, projecting into a cooperating conical recess (18) on the head, characterized in that the longitudinal axis (15) of the second conical plug (14) has a parallel offset (v) with respect to the longitudinal axis (13) of the first conical plug (12).

2. Femoral part according to Claim 1, characterized in that the connecting part (10) is provided with attachment devices (16) for fixing the connecting part (10) relative to the shaft (2) so it cannot rotate.

3. Femoral part according to Claim 2, characterized in that the attachment devices (16) comprise at least one attachment on the connecting part (10) that projects in a form-fitting manner into a matching recess (6) in a given relative position of the shaft and the connecting part.

4. Femoral part according to Claim 2, characterized in that the attachment devices have at least one attachment on the shaft (2) which is in contact in a form-fitting manner with the connecting part (10) in a given relative position of the shaft (2) and the connecting part (10).

5. Femoral part according to Claim 1, characterized in that the first conical plug has a non-round cross section, and the recess (8) in the shaft (2) has a matching non-round cross section.

6. Femoral part according to one of Claims 2 to 5, characterized in that the attachment devices have a screw for securing the connecting part (10) to the shaft (2).

7. Femoral part according to Claim 2, characterized in that the attachment (16) is designed as a polygon between the first and second conical plugs (12, 14) and rests in a polygonal recess (6) at the entrance of the conical recess (8) of the shaft (2).

8. Femoral part according to one of the preceding claims, characterized in that the first conical plug (12) and the second conical plug (14) have different lengths.

9. Femoral part according to one of the preceding claims, characterized in that several connecting parts (10) each with a different offset (v) are provided for combining them with a shaft (2) and a head (20) as selected.

10. Femoral part according to one of the preceding claims, characterized in that the longitudinal axis (15) of the second conical plug (14) has a parallel offset (v) with respect to the longitudinal axis (13) of the first conical plug (12).

## Patentansprüche

1. Femoralteil einer Hüftgelenk-Endoprothese, wobei der Teil einen Schaft (2), einen kugelförmigen Kopf (20) und ein Verbindungsteil (10) aufweist, das den Schaft mit dem Kopf verbindet, wobei am proximalen Ende des Schafts eine konische Vertiefung ausgebildet ist, der ein erster konischer Zapfen (12) am schaftnahen Ende des Verbindungsteils zugeordnet ist, und ein zweiter konischer Zapfen (14) am kopfnahen Ende des Verbindungsteils angeordnet ist, der sich in eine entsprechende konische Vertiefung (18) im Kopf erstreckt, dadurch gekennzeichnet, daß die Längsachse (15) des zweiten konischen Zapfens (14) eine Parallelversetzung (v) bezüglich der Längsachse (13) des ersten konischen Zapfens (12) aufweist.

2. Femoralteil nach Anspruch 1, dadurch gekennzeichnet, daß das Verbindungsteil (10) Befestigungsvorrichtungen (16) aufweist, um das Verbindungsteil (10) bezüglich des Schafts (2) zu fixieren, so daß es sich nicht drehen kann.

3. Femoralteil nach Anspruch 2, dadurch gekennzeichnet, daß die Befestigungsvorrichtungen (16) mindestens eine Befestigung am Verbindungsteil (10) aufweisen, die sich in einer vorgegebenen Relativposition des Schafts (2) und des Verbindungsteils (10) formschlüssig in eine entsprechende Vertiefung (6) erstreckt.

4. Femoralteil nach Anspruch 2, dadurch gekennzeichnet, daß die Befestigungsvorrichtungen mindestens eine Befestigung am Schaft (2) aufweisen, die in einer vorgegebenen Relativposition des Schafts (2) und des Verbindungsteils (10) formschlüssig in Kontakt mit dem Verbindungsteil (10) steht.

5. Femoralteil nach Anspruch 1, dadurch gekennzeichnet, daß der erste konische Zapfen einen nicht-runden Querschnitt aufweist und die Vertiefung (8) im Schaft (2) einen entsprechenden nicht-runden Querschnitt hat.

6. Femoralteil nach einem der Ansprüche 2 bis 5, dadurch gekennzeichnet, daß die Befestigungsvorrichtungen eine Schraube aufweisen, um das Verbindungsteil (10) am Schaft (2) zu befestigen.

7. Femoralteil nach Anspruch 2, dadurch gekennzeichnet, daß die Befestigung (16) als Vieleck zwischen dem ersten und dem zweiten konischen Zapfen (12, 14) ausgebildet ist und in einer vieleckigen Vertiefung (6) am Eingang der konischen Vertiefung (8) des Schafts (2) angeordnet ist.

8. Femoralteil gemäß einem der vorangehenden Ansprüche, dadurch gekennzeichnet, daß der erste konische Zapfen (12) und der zweite konische Zapfen (14) verschiedene Längen haben.

9. Femoralteil gemäß einem der vorangehenden Ansprüche, dadurch gekennzeichnet, daß mehrere Verbindungsteile (10) mit jeweils einer anderen Versetzung (v) bereitgestellt werden, um sie mit einem ausgewählten Schaft (2) und einem ausgewählten Kopf (20) zu kombinieren.

10. Femoralteil gemäß einem der vorangehenden Ansprüche, dadurch gekennzeichnet, daß die Längsachse (15) des zweiten konischen Zapfens (14) eine Parallelversetzung (v) bezüglich der Längsachse (13) des ersten konischen Zapfens (12) aufweist.

## Revendications

1. Partie fémorale d'une endoprothèse d'articulation de la hanche, ladite partie comportant un arbre (2), une tête sphérique (20) et une partie de connexion (10) reliant l'arbre à la tête, un évidement conique étant réalisé à l'extrémité proximale de l'arbre, un premier plot conique de coopération (12) à l'extrémité de la partie de connexion la plus proche de l'arbre, et un deuxième plot conique (14) à l'extrémité de la partie de connexion la plus proche de la tête, faisant saillie dans un évidement conique de coopération (18) sur la tête, caractérisée en ce que l'axe longitudinal (15) du deuxième plot conique (14) a un décalage parallèle (v) relativement à l'axe longitudinal (13) du premier plot conique (12).

2. Partie fémorale selon la revendication 1, caractérisée en ce que la partie de connexion (10) est pourvue de dispositifs d'attachement (16) pour fixer la partie de connexion (10) relativement à l'arbre (2) de façon qu'elle ne puisse pas tourner.

3. Partie fémorale selon la revendication 2, caractérisée en ce que les dispositifs d'attachement (16) comportent au moins un attachement sur la partie de connexion (10) qui fait saillie par une adaptation des formes dans un évidement apparié (6) dans une position relative donnée de l'arbre et de la partie de connexion.

4. Partie fémorale selon la revendication 2, caractérisée en ce que les dispositif d'attachement ont au moins un attachement sur l'arbre (2) qui est en contact par une adaptation des formes avec la partie de connexion (10) dans une position relative donnée de l'arbre (2) et de la partie de connexion (10).

5. Partie fémorale selon la revendication 1, caractérisée en ce que le premier plot conique a une section transversale non-ronde, et l'évidement (8) dans l'arbre (2) a une section transversale non-ronde appariée.

6. Partie fémorale selon l'une des revendications 2 à 5, caractérisée en ce que les dispositif d'attachement ont une vis pour fixer la partie de connexion (10) à l'arbre (2).

7. Partie fémorale selon la revendication 2, caractérisée en ce que l'attachement (16) est conçu comme polygone entre les premier et second plots coniques (12,14) et repose dans un évidement polygonal (6) à l'entrée de l'évidement conique (8) de l'arbre (2).

8. Partie fémorale selon l'une des revendications précédentes, caractérisée en ce que le premier plot conique (12) et le second plot conique (14) ont des longueurs différentes.

9. Partie fémorale selon l'une des revendications précédentes, caractérisée en ce que plusieurs parties de connexion (10), chacune avec un décalage différent (v) sont prévues pour les combiner avec un arbre (2) et une tête (20), selon le choix.

10. Partie fémorale selon l'une des revendications précédentes, caractérisée en ce que l'axe longitudinal (15) du second plot conique (14) a un décalage parallèle (v) relativement à l'axe longitudinal (13) du premier plot conique (12).
